# EUROPÄISCHE PATENTSCHRIFT

(11) **EP 2 179 746 B1**
(45) Veröffentlichungstag und Bekanntmachung des Hinweises auf die Patenterteilung: **09.02.2011**
(21) Anmeldenummer: 09012567.5
(22) Anmeldetag: 05.10.2009
(51) Int. Cl.: A61L 2/26, A61B 19/02

(54) **Behälter zum Sterilisieren und/oder Lagern von medizinischen Gegenständen**
Container to sterilise and/or store medical objects
Récipient de stérilisation et/ou de stockage d'objets médicaux

(30) Priorität: 27.10.2008 DE 102008053301
(43) Veröffentlichungstag der Anmeldung: 28.04.2010
(73) Patentinhaber: MEDICON EG CHIRURGIEMECHANIKER-GENOSSENSCHAFT, 78532 Tuttlingen (DE)
(72) Erfinder: Burger, Andreas, 78532 Tuttlingen (DE); Ruf, Thorsten, 78588 Denkingen (DE)
(74) Vertreter: Mammel, Ulrike

(56) Entgegenhaltungen:
- WO-A1-01/62303
- DE-U1- 20 105 328
- DE-U1- 29 812 996
- US-A- 4 671 943
- US-A1- 2004 256 268
- US-A1- 2004 256 270

## Beschreibung

Die vorliegende Erfindung betrifft einen Behälter beziehungsweise Container zum Sterilisieren und/oder Lagern von medizinischen Instrumenten und Geräten für die Langzeit-Sterilität.

Solche Behälter bestehen üblicherweise aus einem Behälterunterteil und einem Behälterdeckel, die über eine die Langzeit-Sterilität gewährleistende Dichtung fest miteinander verschließbar sind. Am Deckel, an den Seitenwänden oder am Boden des Behälters sind im Durchtrittsbereich Öffnungen vorgesehen, die mit einem auswechselbaren Filter abgedeckt werden, so dass von außen in den Behälter eindringende Luft/Gas oder Dampf den sterilen Filter durchqueren muss.

Um die für die Patienten lebenswichtige Sterilität in dem Behälter zu gewährleisten muss sichergestellt sein, dass kein Durchtritt von (keimhaltiger) Luft, Gas oder Dampf durch die Öffnungen oder die Behälterwand in den Behälter ohne Durchtritt durch den Filter erfolgen kann.

Aus dem Stand der Technik sind Sterilisierbehälter mit einem Deckel und einem Behälterunterteil aus Edelstahl oder Aluminium bekannt, die im Behälterdeckel ein Lochblech umfassen. Von innen ist auf diesem Lochblech über einen Stift eine metallene Scheibe mit größeren Öffnungen aufgeclipst, die der Befestigung und der Abdeckung des Filtermaterials dient. Das Filtermaterial wird durch die Scheibe gegen das Lochblech gepresst.

Bei diesem Sterilisiercontainer kommt es infolge der großen fensterartigen Öffnungen in der Scheibe teilweise zu Verletzungen des darunter liegenden Filterpapiers mit der Folge, dass kontaminierte Luft beispielsweise durch Risse in dem Filtermaterial direkt in den Behälter gelangen kann.

Weiterhin nachteilig ist bei diesem Sterilisierbehälter, dass sich Gegenstände unterhalb der Scheibe verklemmen und die zentrisch befestigte Scheibe einschließlich des Filterpapiers anheben oder nach außen verbiegen können, so dass wiederum ein unerwünschter Durchgang von außen in den Sterilisierbehälter geschaffen wird.

Weiterhin sind Sterilisierbehälter aus Kunststoff bekannt. Im Deckel ist wiederum ein kreisförmiger Durchtrittsbereich mit Öffnungen in das Behälterinnere vorgesehen. Über dem Bereich der Öffnungen ist an der Außenseite des Behälterdeckels ein als Filteraufnahme dienender Kunststoffring befestigt, die Befestigung der Filteraufnahme erfolgt über vier an dem Deckel befestigte Stifte.

In der ringförmigen Filteraufnahme befindet sich der Filter, der an seiner Oberseite von einer kreisförmigen Abdeckscheibe aus Kunststoff mit einer Vielzahl von kleinen Öffnungen abgedeckt wird. Der Deckel ist auf die Filteraufnahme aufgeclipst.

Bei diesem Sterilisiercontainer schützt die auf dem Filtermaterial von außen aufliegende Abdeckscheibe mit den vielen kleinen Öffnungen das Filtermaterial gegen mechanische Eingriffe wie Zerreißen.

Nachteilig ist jedoch, dass der Kunststoffring, beispielsweise wenn sich zwischen dem Ring und dem Behälterdeckel ein Gegenstand verklemmt, ebenfalls angehoben werden kann und - nachdem sich der Filter innerhalb des Rings befindet - ebenfalls ein "filterfreier" Durchgang von außen in das Innere des Sterilisierbehälters geschaffen wird.

Auch bildet sich bei diesem Behälter vermehrt Kondenswasser im Behälterdeckel und in der Abdeckscheibe, so dass das gewünschte Trocknungsergebnis des Behälters nicht erreicht wird.

Aus der US 4,783 321 ist ein Sterilisierbehälter bekannt, bei dem ein spezielles Filterpapier mit Löchern zur Positionierung in Aufnahmepins zwischen dem Behälterboden und einer zweiteiligen Abdeckung durch eine Aufstecken und Drehen in Verschlussposition befestigt wird. Nachteilig ist an diesem Behälter, dass das Filterpapier durch Quetschen und auf das Filterpapier wirkende Rotationskräfte beschädigt wird. Auch ist die Sterilisationsvorrichtung aufwändig aufgebaut und erfordert zudem den Einsatz von speziellem Filterpapier.

Die EP 1 563 854 A1 lehrt einen Sterilisierbehälter, der ebenfalls eine ringförmige Filteraufnahme und eine Abdeckung aufweist und bei dem die Filteraufnahme und die Abdeckung an dem Behälterdeckel durch Renkverschlussbolzen befestigt werden. Auch hier besteht das Problem, dass sich Gegenstände zwischen der Filteraufnahme und der Behälterwand verklemmen und/oder die Filteraufnahme von der Behälterwand angehoben werden kann.

Die Aufgabe der vorliegenden Erfindung besteht darin, einen Behälter zum Sterilisieren und zum Lagern von sterilisierten medizinischen Instrumenten und Geräten vorzuschlagen, bei dem die Gefahr einer direkten Verbindung nach außen und damit eine Kontamination des Sterilguts vermindert wird.

Diese Aufgabe wird bei einem Behälter, der einen Deckel, ein Behälterunterteil, eine Filteraufnahmevorrichtung und eine Filterabdeckung aufweist und der in der Behälterwand einen Bereich mit Durchtrittsöffnungen für Gas/Luft oder Dampf im Deckel und/oder im Behälterunterteil umfasst, dadurch gelöst, dass in der Behälterwand eine den Bereich mit den Durchtrittsöffnungen vollständig umgebende Vertiefung bzw. Nut vorgesehen ist, in welche eine Befestigungseinrichtung der Filteraufnahmevorrichtung mit wenigstens einem Verankerungselement eingreift, insbesondere kann in die Vertiefung/Nut ein Verankerungselement aus Kunststoff als Befestigungseinrichtung eingespritzt sein.

Durch die erfindungsgemäße Befestigung der Filteraufnahme mittels der umlaufenden Nut und der Verankerungselemente wird die Behälterwand nicht durchquert und die Befestigungseinrichtung der Filteraufnahmevorrichtung greift an dem Wandabschnitt der Behälterwand nur an oder ein. Es wird somit vermieden, dass infolge einer Lockerung oder Entfernung der Befestigungseinrichtung wie Stiften, Bolzen oder Schrauben, alternder Dichtungen oder sonstigen Beschädigungen überhaupt ein Durchgang durch die Wand des Sterilcontainers geschaffen wird, durch den dann kontaminierte Luft in den Sterilisiercontainer gelangen kann.

Durch die vollständig umlaufende Nut/Vertiefung, die beispielsweise in die Behälterwand eingefräst sein kann und die in die Nut/Vertiefung eingreifenden Verankerungsmittel, die vorzugsweise ebenfalls vollständig umlaufend ausgebildet sind, wird eine vollständig geschlossene, dichte Abdichtung zwischen Filteraufnahme und Behälterwand erreicht. Diese umlaufende dichte Befestigung der Filteraufnahme verhindert, dass die Filteraufnahme versehentlich angehoben oder sich Gegenstände zwischen Filteraufnahme und Deckel verklemmen können, wie es bei den bislang bekannten Behältern der Fall ist.

Die erfindungsgemäße, die Behälterwand nicht durchdringende Verbindung zwischen der Behälterwand und der Filteraufnahmevorrichtung kommt somit ohne Beschädigung der Außenfläche der Behälterwand beziehungsweise der Außenfläche des Wandabschnitts im Bereich der Durchtrittsöffnungen aus.

Um eine Kontamination zu vermeiden ist die Behälterwand im Bereich der Durchtrittsöffnungen erfindungsgemäß bis auf die Durchtrittsöffnungen auch frei von weiteren Öffnungen oder von die Behälterwand durchquerenden Befestigungs- oder sonstigen Mitteln.

Mittels der Befestigungseinrichtung wird eine Auflagefläche der Filteraufnahmevorrichtung anliegend an den Wandabschnitt der Behälterwand positioniert, so dass das aus dem Stand der Technik bekannte unerwünschte An- oder Abheben der Filteraufnahmevorrichtung einschließlich des darin befindlichen Filters von dem Wandabschnitt oder der Wand des Sterilisiercontainers vermieden wird.

Unter einer an dem Wandabschnitt anliegenden Positionierung wird im Rahmen der vorliegenden Erfindung eine jede Positionierung verstanden, bei der die Auflagefläche der Filteraufnahmevorrichtung an dem Wandabschnitt selbst unter der Wirkung einer die Filtervorrichtung von dem Wandabschnitt abhebenden Kraft, die beispielsweise bei dem im Stand der Technik bekannten Verklemmen wirken kann, weiterhin im Wesentlichen an dem Wandabschnitt anliegt.

Die anliegende Positionierung wird erfindungsgemäß durch die aus Nut/Vertiefung und Verankerungselementen bestehende Befestigungseinrichtung verwirklicht, die Verankerungselemente greifen abdichtend in die Nut ein.

Die Befestigungseinrichtung bestehend aus der Nut und den Verankerungselementen, die beispielsweise durch Einspritzen von Kunststoffmaterial in die Nut hergestellt werden, sind wegen der einfachen Herstellung und der erzielten flächigen, festen Verbindung besonders bevorzugt, insbesondere, wenn T- oder L-förmige Nuten in die Behälterwand eingefräst sind. Prinzipiell könnte in der Nut auch ein metallisches Verankerungselement befestigt werden.

Ebenso ist es möglich, dass mehrere die Durchtrittsöffnungen vollständig umgebende Nuten vorgesehen sind.

Um einen Schutz des Filters durch mechanische Eingriffe von außen zu verhindern, ist der in der Filteraufnahmevorrichtung befindliche Filter nach außen mit einer Filterabdeckung abgedeckt. Vorzugsweise ist die vollständig abnehmbare Filterabdeckung durch lösbare Befestigungsmittel, wie Rast- und/oder Gelenkverbindungen, auf der Filteraufnahmevorrichtung aufgeclipst oder anderweitig befestigt.

Die Filterabdeckung weist eine Vielzahl von kleineren, vorzugsweise kreisförmigen, Öffnungen auf, die so klein sind, dass im Gegensatz zu der eingangs beschriebenen bekannten Filterabdeckung mit großen Öffnungsfenstern eine mechanische Beschädigung oder eine Zerstörung des Filterpapiers durch diese kleinen kreisförmigen Öffnungen vermieden wird.

Um den erforderlichen Durchfluss durch die Durchtrittsöffnungen zu gewährleisten, weisen der Durchtrittsbereich in der Wand und die Filterabdeckung eine Vielzahl dieser kleinen Durchtrittsöffnungen auf. Zur Verbesserung des Durchtritts sind jeweils die Öffnungen in der Filterabdeckung und die Öffnungen in dem Durchtrittsbereich in der Wand entlang einer gemeinsamen Achse ausgerichtet.

Zu einer weiteren Verbesserung der Abdichtung selbst im Falle des Anhebens der Filterabdeckung und Vermeidung eines unerwünschten Durchtritts von kontaminierter Luft/Gas/Dampf tragen weiterhin Dichtlippen bei, die an der Filteraufnahmevorrichtung und/oder der Filterabdeckung und/oder der Behälterwand angeordnet sein können. Der innere Umfang der rahmenförmigen Filteraufnahme ist geringfügig größer als der Außenumgang des Filterpapiers, so dass das Filterpapier innerhalb der rahmenförmigen Filteraufnahme zu Liegen kommt. Das Filterpapier ist an seinem Außenumfang durch eine um den äußeren Filterpapierumfang umlaufende Dichtung, die sich zwischen der Unterseite der Filterabdeckung und der Behälterwand erstreckt und die sich an die Dichtung anschließende, umlaufende Seitenwand der Filteraufnahmevorrichtung begrenzt.

Die Größe der Filteraufnahmevorrichtung ist im Allgemeinen so zu bemessen, dass Standardfilter verwendet werden können. Der Außenrand des Filterpapiers wird von der Innenwand der Filteraufnahme vollständig umschlossen und das Filterpapier ist gegen Verrutschen geschützt. Zusätzliche aus dem Stand der Technik bekannte Positionierlöcher und -pins oder aufwändige, von der runden Form abweichende Filterpapierformen sind somit nicht erforderlich. Die Dichtung bzw. der Dichtungsring grenzt seitlich an dem Verankerungselement der Filteraufnahmevorrichtung.

Eine Dichtung zwischen der Filteraufnahme und dem Behälterboden ist nicht erforderlich, da die Befestigungseinrichtung der Filteraufnahme in den Behälterboden abdichtend eingreift und außerhalb des Bereichs mit den Durchtrittsöffnungen durch die Behälterwand kein Durchtritt erfolgt.

Vorzugsweise ist die Filteraufnahme, in der der Filter gehalten wird, ringförmig, das Filterpapier rund und der Außendurchmesser des Filterpapiers geringfügig kleiner als der Innendurchmesser der ringförmigen Filteraufnahme. Auch in diesem Fall befindet sich zwischen dem Außenumfang des Filterpapiers und dem Innenumfang der Filteraufnahme eine Dichtung, die in diesem Fall rund ist und das Filterpapier vollständig umschließt.

Das Filterpapier liegt weiterhin vollflächig auf der Behälterwand mit den Durchtrittsöffnungen auf. Oberhalb des Filterpapiers befindet sich die Filterabdeckung, mittels der das Filterpapier von oben gegen die Behälterwand und die Öffnungen gepresst wird.

Vorzugsweise ist zwischen der Unterseite der Filterabdeckung und der Behälterwand ebenfalls eine Dichtung, insbesondere ein Dichtring, vorgesehen.

Vorzugsweise ist die Filterabdeckung an der Filteraufnahme derart befestigt, dass beim Öffnen und Schließen der Filterabdeckung auf das Filterpapier nur oder im Wesentlichen nur Kräfte senkrecht zur Ebene des Filterpapiers wirken und so die in der Papierebene wirkenden Kräfte, die zu einem Einreißen oder Zerreißen des Filterpapiers führen können, wie beispielsweise durch eine Rotationsbewegung auf den Deckel, vermieden werden.

Dadurch, dass die Filteraufnahmevorrichtung mittels der Verankerungselemente abdichtend mit der Behälterwand befestigt ist und die Filterabdeckung wiederum an der Filteraufnahmevorrichtung befestigt ist, ist nur eine Abdichtung der Filterabdeckung gegenüber der Filteraufnahme und eine Abdichtung der Filterabdeckung gegenüber der Behälterwand erforderlich, um die gewünschte Dichtigkeit und Sicherheit zu erreichen.

Eine Wesentlichen nur senkrecht zur Ebene des Filterpapiers wirkende Kraft beim Öffnen und Schließen der Filterabdeckung kann durch eine aufklappbare oder durch Befestigung an zwei oder mehr lösbaren Befestigungselementen lösbare Filterabdeckung ermöglicht werden.

Dadurch, dass die Filterabdeckung, die aus einer den Filter abdeckenden, scheibenförmigen Abdeckung besteht, nur zum Schutz und zur Abdeckung des Filters und nicht auch zur Befestigung am Behälterboden dient, ist ein einfacher, einteiliger Aufbau möglich und komplizierte Befestigungen und Drehbewegungen auf das Filterpapier werden vermieden. Das Filterpapier ist in der erfindungsgemäßen Vorrichtung durch den umlaufenden Rand der Filteraufnahmevorrichtung und die beiden das Filterpapier oben und unten begrenzenden planen Flächen, nämlich dem Behälterboden und der Filterabdeckung, fixiert.

In einer bevorzugten Variante sind das Behälterunterteil und der Behälterdeckel aus Metall, insbesondere aus Aluminium oder Edelstahl, die Filteraufnahmevorrichtung und die Filterabdeckung aus Kunststoff, insbesondere aus PPSU, ausgebildet.

Durch diese Metall/Kunststoff-Kombination wird durch den metallischen Behälter eine gute Wärmeleitung erzielt und somit auch eine gute Trocknung erreicht, die durch den verhältnismäßig geringen Kunststoffanteil in der Filteraufnahme und der Filterabdeckung nicht wesentlich beeinträchtigt wird.

Die Filteraufnahme und Filterabdeckung sind als Spritzgussteil einfach herstellbar, und es kann durch die Kombination Metall/Kunststoff die gewünschte flächige Verbindung ohne Beeinträchtigung der Oberfläche des Behälters und ohne Durchbrechung der Behälteroberfläche mit weiteren Kontaminationsrisiken erzielt werden.

Die Erfindung wird nachfolgend anhand von Ausführungsbeispielen näher beschreiben. Es zeigen:
- Figur 1: einen Ausschnitt aus einer Außenwand eines Sterili- sierbehälters mit dem Bereich der Durchtrittsöff- nungen und der Filteraufnahmevorrichtung,
- Figur 2: eine Draufsicht auf die Filteraufnahmevorrichtung,
- Figur 3: einen Schnitt entlang der Linie A-A durch die Filter- aufnahmevorrichtung aus Figur 2,
- Figur 4: eine Draufsicht auf die Filterabdeckung,
- Figur 5: einen Schnitt entlang der Linie B-B durch die Filter- abdeckung aus Figur 4 und
- Figur 6: einen Schnitt durch einen Ausschnitt einer Behäl- terwand mit Filteraufnahmevorrichtung, Filter und Filterabdeckung.

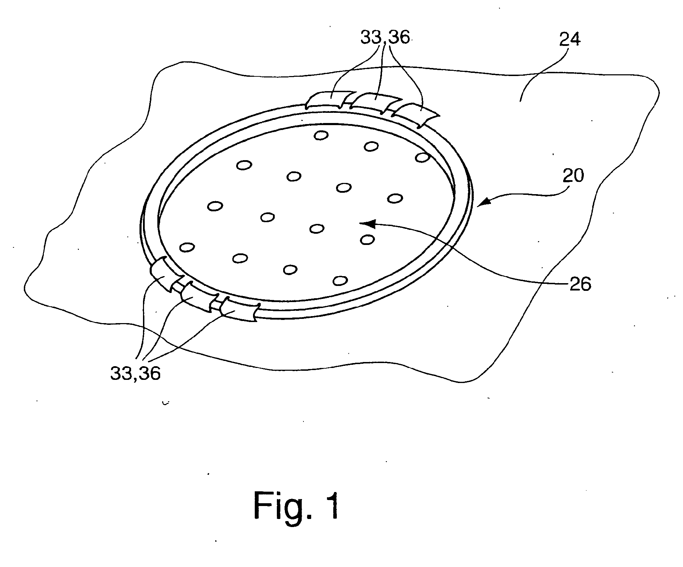

Figur 1 zeigt einen Ausschnitt aus der Behälterwand 24 eines Sterilisierbehälters mit einem Bereich 26 mit Öffnungen 27 zum Durchtritt von Luft/Gas oder Dampf. Der Durchtrittsbereich 26 in der Behälterwand 24 kann sich im Deckel, in der Seitenwand oder auch im Boden des Sterilisierbehälters befinden.

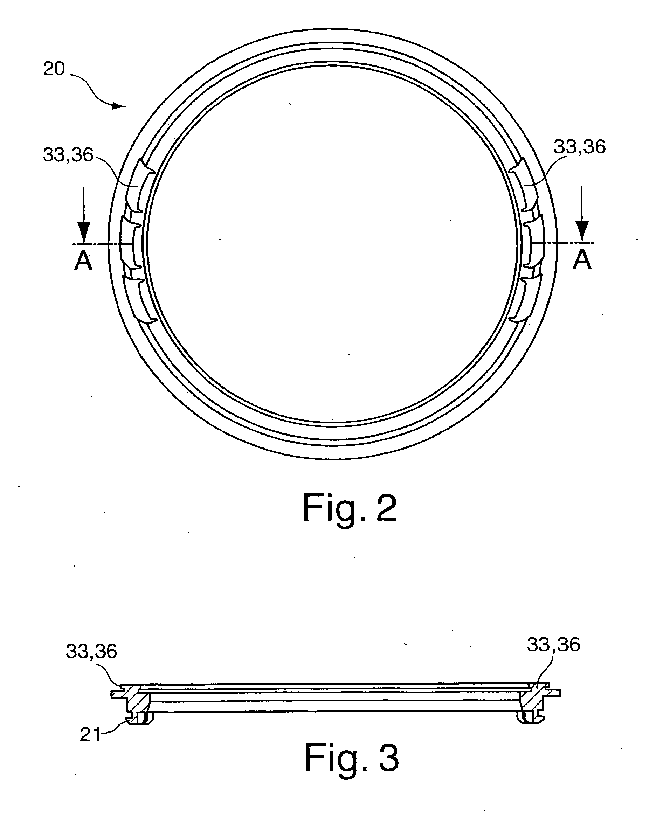

Der Durchtrittsbereich 26 mit den Öffnungen 27 ist von der ringförmigen Filteraufnahmevorrichtung 20 vollständig umgeben. Die Filteraufnahmevorrichtung 20 (Figuren 2, 3 und 6) ist mittels Spritzguss hergestellt und in dem Wandabschnitt 25 der Wand 24 dadurch befestigt, dass der Kunststoff in die kreisförmig den Durchtrittsbereich 26 umgebende T-Nut 32 in dem Wandabschnitt 25 direkt eingespritzt wird. Hierdurch entstehen Verankerungselemente 21, die die T-Nut 32 hintergreifen, so dass eine flächige, das Filterpapier vollständig umgebende, kreisförmig verlaufende, dichte Verbindung erzielt wird.

Durch diese Befestigung wird auch die Auflagefläche 22 der Filteraufnahmevorrichtung 20 zu dem Wandabschnitt 25 durchgehend anliegend positioniert, so dass es nicht möglich ist, die Filteraufnahmevorrichtung 20 von dem Wandabschnitt 25 abzuheben und so einen unerwünschten Durchtritt in den Behälter zu ermöglichen.

Um eine Kontamination zu verhindern, ist ein Filter 44, insbesondere ein flächiger Papierfilter, der auf dem Durchtrittsbereich 26 aufliegt, innerhalb der Filteraufnahme 20vorgesehen. Der Filter 44 wird mittels der Filterabdeckung 40 von oben gegen die Behälterwand 24 und die Öffnungen 27 gedrückt.

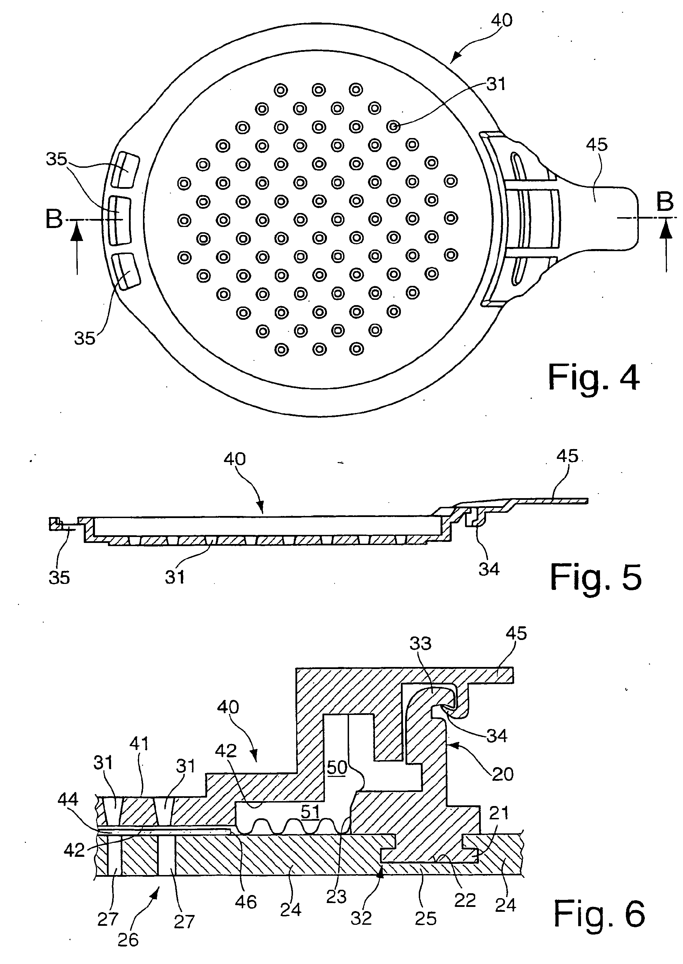

Die Filterabdeckung 40 (Figuren 4, 5) weist ebenfalls Öffnungen 31 auf, die dem Gas-/Dampfdurchtritt dienen. Für eine guten Durchtritt sind die Öffnungen 31 in der Filterabdeckung 40 und die Öffnungen 27 in der Behälterwand 24 vorzugsweise entlang einer gemeinsamen Achse angeordnet, wobei sich die Öffnungen 31 in der Abdeckung 40 von der Außenseite 41 der Abdeckung 40 in Richtung der Innenseite 42 hin verjüngen (vergleiche Figur 6). Der (kleinste) Durchmesser der Öffnungen 31 und 27 beträgt etwa 2,8 mm, die Anzahl der Öffnungen im Durchtrittsbereich 26 und in der Filterabdeckung 40 liegt jeweils bei circa 80 bis 100.

Um die Filterabdeckung 40 gegen den Filter 44 zu pressen und einen seitlichen Durchtritt an/unter dem Filter 44 zu vermeiden, weist die Filterabdeckung 40 zusätzlich eine ringförmige Dichtlippe 51 auf, die an der nach unten gerichteten Innenseite 42 der Filterabdeckung 40 verläuft und die die nach außen weisende Kante 46 des Filters 44 seitlich umgibt.

Weiterhin wird ein seitlicher Durchtritt dadurch vermieden, dass die Filterabdeckung 40 seitlich gegenüber der nach innen gerichteten Seitenwand 23 der Filteraufnahmevorrichtung 20 durch ringförmig umlaufende Dichtlippen 50 seitlich abgedichtet ist.

Somit ist selbst im Falle eines leichten Anhebens der Filterabdeckung 40 die erforderliche Dichtigkeit gewährleistet, und ein seitlicher Durchtritt an/unter den Filter wird vermieden.

Um den Filter 44 auszuwechseln, ist die Abdeckung 40 an der Filteraufnahmevorrichtung 20 durch eine lösbare Verbindung, beispielsweise durch Rastverbindungen 33, 34 und lösbare Gelenkverbindungen 35, 36, befestigt.

Zur Verbesserung der Bedienbarkeit der Filterabdeckung 40 kann diese auch mit einem Griffelement 45 versehen sein.

Die Filterabdeckung 40 und die Filteraufnahmevorrichtung 20 sind aus Kunststoff, insbesondere aus PPSU, und die Dichtungen 50 und 51 aus Silikon (LSR) ausgebildet.

Das Behälterunterteil 17 und der Deckel sind aus Aluminium oder Edelstahl.

## Patentansprüche

1. Behälter zum Sterilisieren und/oder zum Lagern von sterilisierten medizinischen Instrumenten,
mit einem Deckel, einem Behälterunterteil, einer Filteraufnahmevorrichtung (20) und einer Filterabdeckung (40), wobei der Behälter in der Behälterwand (24) einen Bereich (26) mit mehreren Durchtrittsöffnungen (27) für Gas/Luft oder Dampf im Deckel und/oder im Behälterunterteil aufweist,
**dadurch gekennzeichnet,**
**dass** in der Wand (24) eine den Bereich (26) mit den Durchtrittsöffnungen (27) vollständig umgebende Vertiefung/Nut (32) vorgesehen ist, in welche eine Befestigungseinrichtung (21, 32) der Filteraufnahmevorrichtung (20) mit wenigstens einem Verankerungselement (21) eingreift.

2. Behälter nach Anspruch 1, **dadurch gekennzeichnet, dass** in die Vertiefung/Nut (32) ein Verankerungselement (21) aus Kunststoff als Befestigungseinrichtung eingespritzt ist.

3. Behälter nach Anspruch 1 oder 2, **dadurch gekennzeichnet, dass** die Befestigungseinrichtung (21, 32) der Filteraufnahmevorrichtung (20) an oder in einem Wandabschnitt (25) der Behälterwand (24) an- oder eingreift und eine Auflagefläche (22) der Filteraufnahmevorrichtung (20) zu dem Wandabschnitt (25) anliegend positioniert.

4. Behälter nach einem der vorangegangenen Ansprüche, **dadurch gekennzeichnet, dass** die Verankerungselemente (21) abdichtend in die Nut (32) eingreifen.

5. Behälter nach einem der vorangegangenen Ansprüche, **dadurch gekennzeichnet, dass** der Bereich (26) der Durchtrittsöffnungen (27) bis auf die Durchtrittsöffnungen (27) frei von weiteren Öffnungen oder die Behälterwand (24) durchquerenden Befestigungs- oder sonstigen Mitteln ist.

6. Behälter nach einem der vorangegangenen Ansprüche, **dadurch gekennzeichnet, dass** die Filteraufnahmevorrichtung (20) rahmenförmig ist und zur Aufnahme eines Filters innerhalb des Rahmens dient.

7. Behälter nach einem der vorangegangenen Ansprüche, **dadurch gekennzeichnet, dass** das Verankerungselement (21) und die Nut (32) ringförmig geschlossen und abdichtend ausgebildet sind.

8. Behälter nach einem der vorangegangenen Ansprüche, **dadurch gekennzeichnet, dass** die Verankerungselemente (21) aus Kunststoff oder Metall sind.

9. Behälter nach einem der vorangegangenen Ansprüche, **dadurch gekennzeichnet, dass** die Nut (32) eine T-Nut oder eine L-Nut ist.

10. Behälter nach einem der vorangegangenen Ansprüche, **dadurch gekennzeichnet, dass** die Filteraufnahmevorrichtung (20) Mittel (33, 36) zur Befestigung der Filterabdeckung (40) sowie eine Vielzahl von Öffnungen (31) für den Luft-/Gas-/Dampfdurchtritt aufweist.

11. Behälter nach Anspruch 10, **dadurch gekennzeichnet, dass** die Öffnungen (31) in der Filterabdeckung (40) und die Öffnungen (27) in dem Durchtrittsbereich (26) in der Wand (24) jeweils entlang einer gemeinsamen Achse angeordnet sind.

12. Behälter nach einem der vorangegangenen Ansprüche, **dadurch gekennzeichnet, dass** an der Unterseite der Filterabdeckung (40) eine ringförmige Dichtung (51) zur Abdichtung gegenüber der Behälterwand (24) vorgesehen ist.

13. Behälter nach einem der Ansprüche 8 bis 11, **dadurch gekennzeichnet, dass** eine Dichtung (50) zwischen der Filterabdeckung (40) und der nach innen gerichteten Seitenwand (23) der Filteraufnahmevorrichtung (20) vorgesehen ist.

14. Behälter nach einem der Ansprüche 1 bis 13, **dadurch gekennzeichnet, dass** der Deckel und das Behälterunterteil aus Metall, insbesondere aus Aluminium oder Edelstahl, sind.

15. Behälter nach einem der Ansprüche 1 bis 14, **dadurch gekennzeichnet, dass** die Filteraufnahmevorrichtung (20) und die Filterabdeckung (40) aus Kunststoff, insbesondere aus PPSU, sind.

## Claims

1. A container for sterilising medical instruments and/or for storing sterilised medical instruments, comprising a lid, a container base, a filter mounting appliance (20) and a filter cover (40), said container having a zone (26) formed in the container wall (24) in the region of the lid and/or the container base and provided with a plurality of through holes (27) for the passage of gas/air or vapour, **characterised in that** the wall (24) is provided with a depression/groove (32) which surrounds the zone (26) provided with the through holes (27) in its entirety and with which a fastening device (21, 32) of the filter mounting appliance (20) engages by means of at least one anchoring element (21).

2. The container as claimed in claim 1, **characterised in that** an anchoring element (21) made of plastic and serving as a fastening device is inserted into the depression/groove (32) by way of injection moulding.

3. The container as claimed in claim 1 or 2, **characterised in that** the fastening device (21, 32) of the filter mounting appliance (20) is applied to, or engages with, a wall portion (25) of the container wall (24) and positions a contact surface (22) of the filter mounting appliance (20) in close contact with the wall portion (25).

4. The container as claimed in any of the preceding claims, **characterised in that** the anchoring elements (21) sealingly engage with the groove (32).

5. The container as claimed in any of the preceding claims, **characterised in that** the zone (26) provided with the through holes (27) is not provided, apart from said through holes (27), with any other openings or with fastening means or other means extending through the container wall (24).

6. The container as claimed in any of the preceding claims, **characterised in that** the filter mounting appliance (20) is shaped in the form of a frame and serves for accommodating a filter within said frame.

7. The container as claimed in any of the preceding claims, **characterised in that** the anchoring element (21) and the groove (32) are each annularly closed and in sealing engagement with each other.

8. The container as claimed in any of the preceding claims, **characterised in that** the anchoring elements (21) are made of plastic or metal.

9. The container as claimed in any of the preceding claims, **characterised in that** the groove (32) is a T-shaped groove or an L-shaped groove.

10. The container as claimed in any of the preceding claims, **characterised in that** the filter mounting appliance (20) is provided with means (33, 36) for fastening the filter cover (40) and with a plurality of openings (31) for the passage of air/gas/vapour therethrough.

11. The container as claimed in claim 10, **characterised in that** the openings (31) in the filter cover (40) and the openings (27) in the throughpassage zone (26) of the wall (24) are respectively arranged along a common axis.

12. The container as claimed in any of the preceding claims, **characterised in that** the lower surface of the filter cover (40) is provided with an annular gasket (51) for sealing with respect to the container wall (24).

13. The container as claimed in any one of claims 8 to 11, **characterised in that** a gasket (50) is provided between the filter cover (40) and the inwardly-facing lateral wall (23) of the filter mounting appliance (20).

14. The container as claimed in any one of claims 1 to 13, **characterised in that** the lid and the container base are both made of metal, in particular of aluminium or stainless steel.

15. The container as claimed in any one of claims 1 to 14, **characterised in that** the filter mounting appliance (20) and the filter cover (40) are made of plastic, in particular of PPSU.

## Revendications

1. Récipient permettant de stériliser des instruments médicaux et/ou de stocker des instruments médicaux stérilisés, comprenant un couvercle, une partie inférieure de récipient, un dispositif porte-filtre (20) et un couvre-filtre (40), le récipient présentant, au niveau de la paroi de récipient (24), une zone (26) située dans le couvercle et/ou dans la partie inférieure de récipient et comportant plusieurs ouvertures de traversée (27) pour du gaz/de l'air ou de la vapeur, **caractérisé en ce qu'**il est prévu, dans la paroi (24), une dépression/rainure (32) entourant complètement la zone (26) comportant les ouvertures de traversée (27) et dans laquelle engrène un système de fixation (21, 32) du dispositif porte-filtre (20) avec au moins un élément d'ancrage (21).

2. Récipient selon la revendication 1, **caractérisé en ce que** dans la dépression/rainure (32) est réalisé par injection un élément d'ancrage (21) en matière plastique servant de système de fixation.

3. Récipient selon la revendication 1 ou 2, **caractérisé en ce que** le système de fixation (21, 32) du dispositif porte-filtre (20) repose contre ou engrène dans une partie de paroi (25) de la paroi de récipient (24), et positionne une surface d'appui (22) du dispositif porte-filtre (20) de manière ajustée contre la partie de paroi (25).

4. Récipient selon l'une des revendications précédentes, **caractérisé en ce que** les éléments d'ancrage (21) engrènent de manière hermétique dans la rainure (32).

5. Récipient selon l'une des revendications précédentes, **caractérisé en ce que** la zone (26) des ouvertures de traversée (27) est, mises à part les ouvertures de traversée (27), exempte de toute autre ouverture ou de tout autre moyen de fixation ou autre traversant la paroi de récipient (24).

6. Récipient selon l'une des revendications précédentes, **caractérisé en ce que** le dispositif porte-filtre (20) est en forme de cadre et sert à loger un filtre à l'intérieur du cadre.

7. Récipient selon l'une des revendications précédentes, **caractérisé en ce que** l'élément d'ancrage (21) et la rainure (32) sont en forme d'anneau fermé et sont réalisés de manière à fermer hermétiquement.

8. Récipient selon l'une des revendications précédentes, **caractérisé en ce que** les éléments d'ancrage (21) sont en matière plastique ou en métal.

9. Récipient selon l'une des revendications précédentes, **caractérisé en ce que** la rainure (32) est une rainure en T ou une rainure en L.

10. Récipient selon l'une des revendications précédentes, **caractérisé en ce que** le dispositif porte-filtre (20) présente des moyens (33, 36) permettant de fixer le couvre-filtre (40) ainsi qu'une pluralité d'ouvertures (31) pour le passage d'air, de gaz, de vapeur.

11. Récipient selon la revendication 10, **caractérisé en ce que** les ouvertures (31) situées dans le couvre-filtre (40) et les ouvertures (27) situées dans la zone de traversée (26) de la paroi (24) sont respectivement disposées le long d'un axe commun.

12. Récipient selon l'une des revendications précédentes, **caractérisé en ce qu'**un joint d'étanchéité annulaire (51) est prévu sur la face inférieure du couvre-filtre (40) en vue d'assurer l'étanchéité par rapport à la paroi de récipient (24).

13. Récipient selon l'une des revendications 8 à 11, **caractérisé en ce qu'**un joint d'étanchéité (50) est prévu entre le couvre-filtre (40) et la paroi latérale (23) du dispositif porte-filtre (20) dirigée vers l'intérieur.

14. Récipient selon l'une des revendications 1 à 13, **caractérisé en ce que** le couvercle et la partie inférieure de récipient sont en métal, notamment en aluminium ou en acier spécial.

15. Récipient selon l'une des revendications 1 à 14, **caractérisé en ce que** le dispositif porte-filtre (20) et le couvre-filtre (40) sont en matière plastique, notamment en PPSU.
